# EUROPEAN PATENT APPLICATION

(11) **EP 2 404 664 A1**
(43) Date of publication of application: **11.01.2012**
(21) Application number: 11005350.1
(22) Date of filing: 21.06.2005
(51) Int. Cl.: B01D 65/00, B01D 63/02, B01D 61/30, A61M 1/16

(54) **A support for a separation device and extracorporeal blood treatment using said support and separation device**

(30) Priority: 14.07.2004 IT MO20040180
(62) Divisional of application: 05013338.8
(71) Applicant: Gambro Lundia AB, S-220 10 Lund (SE)
(72) Inventor: Dannenmaier, Jürgen, 72336 Baligen (DE); Ertl, Thomas, 72406 Bisingen (DE); Vinci, Luca, 46025 Poggio Rusco (IT); Tonelli, Claudio, 41121 Modena (IT)
(74) Representative: Ponzellini, Gianmarco

(57) **Abstract**

A support comprising a first connection element (21) having a central fluid access port (22) and a peripheral fluid access port (23) which surrounds said central fluid access port (22); and a second connection element (37) having at least a third fluid access port (38). The first connection element (21) and second connection element (22) may assume at least a first operative closed position, in which a separation device (5) arranged between said first connection element (22) and said second connection element (23) in a work position is fluidly connected to all three of said fluid access ports, and at least a second operative open configuration, in which said separation device can be inserted into and disinserted from between said first connection element (21) and said second connection element (22).

## Description

### Background of the Invention

The invention relates to a support for a separation device.

Specifically, though not exclusively, the invention can be usefully applied in an apparatus for extracorporeal blood treatment, for example for one or more of the following treatments: hemodialysis, hemofiltration, hemodiafiltration, pure ultrafiltration, plasmapheresis. In this field, a separation device made according to the present invention can have, for example, the function of a blood treatment unit (dialysis filter), or a filter for cleaning the dialysis liquid (ultrafilter); a support made according to the present invention can advantageously be part of a machine for extracorporeal blood treatment, with the aim of keeping the separation device operatively associated to the machine in the correct operating position.

The prior art comprises various types of filtration device used in dialysis treatment, such as for example filters for dialysis (or dialysers) for extracorporeal blood purification, and ultrafilters used in dialysis machines for on-line preparation of dialysis liquid and infusion liquid.

The majority of the filtration devices at present used for these operations comprise an elongate housing body, containing a bundle of hollow fibres which separate a first fluid space, formed by the internal volume of the fibres, from a second fluid space, comprised between the fibres and the housing body. Each fluid space generally has - at least in the case of a dialyser- two fluid connection ports, one for inlet and the other for outlet. In the case of an ultrafilter the second fluid space can have only one fluid connection port for outlet of the filtered liquid.

In known dialysers the connection ports fluidly connected to the first fluid space are arranged, generally, on two caps which close the ends of the housing body. The central axis of these connection ports can be parallel (see the Gambro dialyser commercially known as Polyflux ®), or perpendicular (see the Gambro dialyser commercially known as Arylane ®).

The connection ports fluidly connected to the second fluid space are generally arranged either on the two end caps (see EP 0 887 100) or on the housing body (the more common configuration). The central axis of the connection ports is normally perpendicular to the longitudinal axis of the housing body. WO 03/028859 teaches a filtration device with the connection ports all arranged on the housing body.

Also known are dialysis machines having a support to which a filtration device can be removably fixed, in a work position in which the device is in fluid communication with a hydraulic circuit internal of the machine. Examples of such supports are taught in EP 0 887 100 and in WO 01/08722.

Dialysers having two perpendicular connection ports and two connection ports parallel to the longitudinal axis of the housing body exhibit some limitations and drawbacks.

Firstly the positioning of the dialyser on the machine, and more precisely the centring of the connection ports of the dialyser on the fluid attachments arranged on the machine requires an extremely high degree of precision when it comes to the relative positions of the connection ports on the filtering device. Since commmonly the housings of these filtration devices are made by injection moulding, the required precision is hard to be achieved and leads to a consequent difficulty in realizing a perfect fluid seal in the coupling zone.

Secondly, owing to the different orientation of the connection ports, the filter support is constructionally complex and expensive.

Also, the mounting of the filter on the dialysis machine requires a relatively long time and needs skilled personnel. As a result it is not easy to set up automatically or semiautomatically.

Filtration devices having all their connection ports perpendicular to the longitudinal axis of the housing body, and arranged on the same side of the filter (see EP 0 887 100) have the problem that stagnant areas of fluid collect, where there is greater probability of accumulation of air bubbles, especially in the second fluid space, the space external of the fibres. This fluid stagnation and the accumulation of air bubbles are undesirable phenomena, especially when attempting to obtain good sterilization and disinfection of the filter.

A solution to the stagnation problem could consist in arranging all the connection ports at the end of the filtration device, each with its own axis orientated parallel to the longitudinal axis of the housing body. This solution, however, would have the same limitation and drawbacks as described herein above: laborious and imprecise positioning of the filter, complexity of the support.

EP 1 340 534 teaches a filtration device having, at one end, two fluid connection openings arranged coaxially, a central opening connected to the first fluid space and another annular peripheral opening connected to the second fluid space, with the common central axis coinciding with the longitudinal axis of the main body of the filtration device. The sealing material, which is arranged at an end of the hollow fibre bundle and which keeps the fibres together, sealedly fluidly separates the second fluid space from a compartment leading into the centrally-located fluid connection opening. However this sealing material (usually polyurethane) has the drawback of being susceptible to breakage, cracking, deformation and other structural imperfections, by effect of the heat stress it is subject to, for example during the steam - or heat - sterilization process. Subsequently there may be loss of seal capacity.

EP 1 340 534 teaches a housing body having at an end thereof a series of indentations afforded in the sealing material. This solution, according to the applicant's explanation, reduces the risk of formation of breakages due to material shrinkage, thus guaranteeing a perfect fluid seal. However this solution too exhibits various limitations and drawbacks. Firstly the manufacturing process of the filtration device made according to this process is relatively complex and expensive. Secondly this type of polyurethane sealing cannot guarantee a fluid seal which is good beyond a certain number of heat-disinfection cycles, since the use of a commercial product which uses this type of potting sealing (Fresenius DiaSafe ®), is limited by its manufacturor to a specific number of disinfection cycles. Further, the seal connection between the fluid connection port, formed by the two coaxial openings, and an appropriate support with an efficient, stable and enduring separation between the two fluid spaces, is not easy to achieve and not shown in EP 1 340 534.

### 1.1.1.1.1 Summary of the Invention ,

In this situation, an aim of the present invention is to provide a separation device which is constructionally simple and economical.

A further aim of the invention is to provide a support on which a separation device can be fixed and removed easily.

An advantage of the invention is that it renders a correct positioning of the separation device in the work position easy.

A further advantage of the invention is that it guarantees sealed fluid separation in the connection zone between the separation device and the support.

A further advantage is the seal reliability of the sealing material at the end of the hollow fibre bundle, even after the material has been subjected to a high number of heat-disinfecting cycles.

A still further advantage is that a separation device is made available which is internally without zones prone to fluid stagnation.

The separation device of the present invention can be rinsed very easily and reliably.

The separation device of the invention is advantageously free of fluid stagnation zones or zones where air bubbles can collect.

These aims and advantages and more besides are all attained by the present invention, as it is characterised in one or more of the appended claims.

According to an embodiment of the invention a first separation device comprises: a housing body (2); a first fluid space (3) in said housing body; a second fluid space (4) situated between said first fluid space (3) and said housing body (2); a membrane separation element (5) separating said first fluid space (3) from said second fluid space (4); at least one sealing element (6) arranged at an end of said membrane separation element (5); at least one first compartment (7) fluidly connected to said first fluid space (3); at least one second compartment (9) fluidly connected with said second fluid space (4) and fluidly isolated from said first compartment (7).

According to a further embodiment, said first compartment (7) is provided with a first access opening (17), said second compartment (9) is provided with a second access opening (18); at least a part of said second access opening (18) surrounds at least a part of said first access opening (17).

According to a further embodiment said first access opening (17) and said second access opening (18) are coaxial to one another.

According to a further embodiment, each of the previous embodiments comprises at least an annular body (12) interpositioned between said second compartment (9) and said second fluid space (4).

According to a further embodiment, each of the previous embodiments comprises at least one passage (13), contiguous to said annular body (12), or passing through said annular body (12), for fluidly connecting said second compartment (9) with said second fluid space (4).

According to a further embodiment, in each of the previous embodiments said at least one passage (13) is afforded in said annular body (12).

According to a further embodiment, in each of the previous embodiments said sealing element (6) has a peripheral surface which is radially directed, with reference to a longitudinal axis (x-x) of said membrane separation element (5), and which is circumferentially contactingly embraced by an annular body (12) interpositioned fluidly between said second compartment (9) and said second fluid space (4).

According to a further embodiment, in each of the previous embodiments said annular body (12) has a closed annular shape.

According to a further embodiment, in each of the previous embodiments said peripheral surface of said sealing element (6) and said annular body (12) have a reciprocal contact zone, which is free of undercuts in a radial direction towards the periphery.

According to a further embodiment, in each of the previous embodiments said sealing element (6) is in contact with an annular body (12) fluidly interpositioned between said second compartment (9) and said second fluid space (4).

According to a further embodiment, in the previous embodiment said contact is detachable.

According to a further embodiment, each of the previous embodiments comprises an annular body (12) which is fluidly interpositioned between said second compartment (9) and said second fluid space (4), said annular body (12) being distinct from said housing body (2).

According to a further embodiment, each of the previous embodiments comprises an annular body (12) fluidly interpositioned between said second compartment (9) and said second fluid space (4), said annular body (12) being interpositioned contactingly between said sealing element (6) and said housing body (2).

According to a further embodiment, in each of the previous embodiments at least a part of said sealing element (6) is a radially extended plate, with reference to a longitudinal axis (x-x) of said membrane separation element (5), said plate of said sealing element (6) having a radially-directed peripheral surface, which is at least partially in contact with an annular body (12) fluidly interpositioned between said second compartment (9) and said second fluid space (4).

According to a further embodiment, in the previous embodiment said annular body (12) and said peripheral surface are in reciprocal contact at least at an axial undercut'(15).

According to a further embodiment, each of the previous embodiments comprises an annular body (12) fluidly interpositioned between said second fluid space (4) and said second compartment (9), at least a part of said annular body (12) radially surrounding said sealing element (6), where radially is intended to mean with reference to a longitudinal axis (x-x) of said membrane separation element (5).

According to a further embodiment, each of the previous embodiments comprises an annular body (12) interpositioned fluidly between said second fluid space (4) and said second compartment (9), at least a part of said annular body (12) radially containing a zone which is not occupied by said second fluid space, where radially is intended to mean with reference to a longtitudinal axis (x-x) of said membrane separation element (5).

According to a further embodiment, each of the previous embodiments comprises at least a first cap (8) conformed and arranged in order to at least partly define said first compartment (7) together with said sealing element (6).

According to a further embodiment, in each of the previous embodiments said first cap (8) has a terminal wall having an external edge which internally delimits said second access opening (18), and an internal edge which externally delimits said first access opening (17).

According to a further embodiment, in each of the previous embodiments said first cap (8) is in contact with said housing body (2).

According to a further embodiment, in each of the previous embodiments said first cap (8) has an annular edge which is coupled to an annular edge of said housing body (2).

According to a further embodiment, in each of the previous embodiments said first cap (8) is solidly connected to said housing body (2).

According to a further embodiment, in each of the previous embodiments said first cap (8) at least partly covers an external side (6b) of said sealing element (6).

The device of any one of claims from 18 to 23, wherein a part of said first cap (8), which is interpositioned between said second compartment (9) and said second fluid space (4), is provided with at least one hole (16) for fluid communication between said second compartment (9) and said second fluid space (4).

According to a further embodiment, in the previous embodiment said at least one hole (16) extends prevalently in an axial direction, with reference to a longitudinal axis (x-x) of said membrane separation element (5).

According to a further embodiment, each of the previous embodiments comprises: at least a first cap (8) conformed and arranged in such a way as to at least partially define said first compartment (7) in collaboration with said sealing element (6); at least a second cap (10) conformed and arranged in such a way as to at least partially define said second compartment (9) in collaboration with said first cap (8).

According to a further embodiment, in the previous embodiment said second cap (10) at least partly covers said first cap (8).

According to a further embodiment, in each of the two previous embodiments said second cap (10) is coaxial to said first cap (8).

According to a further embodiment, in each of the three previous embodiments said second cap (10) is arranged entirely beyond said housing body (2), with reference to a parallel direction to a longitudinal axis (x-x) of said housing body (2).

According to a further embodiment, in each of the four previous embodiments said second cap (10) is in contact with said first cap (8).

According to a further embodiment, in each of the five previous embodiments said second cap (10) has an annular edge which is coupled to an annular edge of said first cap (8).

According to a further embodiment, in each of the six previous embodiments said second cap (10) is solidly connected to said first cap (8).

According to a further embodiment, in each of the previous embodiments said membrane separation element (5) comprises a bundle of hollow fibres.

According to a further embodiment, in each of the previous embodiments said membrane separation element (5) is prevalently extended according to a longitudinal axis (x-x).

According to a further embodiment, in each of the previous embodiments said sealing element (6) comprises a potting material.

According to a further embodiment, in each of the previous embodiments said sealing element (6) has an internal side (6a) which fluidly communicates with said second fluid space (4), and an external side (6b) which fluidly communicates with said first fluid space (3).

According to a further embodiment of the invention a second separation device comprises: a housing body (2); a first fluid space (3) in said housing body (2); a second fluid space (4) situated between said first fluid space (3) and said housing body (2); a membrane separation element (5) which separates said first fluid space (3) from said second fluid space (4); at least one sealing element (6) arranged at an end of said separation element (5); at least a first compartment (7) in fluid communication with said first fluid space (3); at least a second compartment (9) in fluid communication with said second fluid space and fluidly isolated from said first compartment; at least one cap (8) interpositioned between said first compartment (7) and said second compartment (9); at least one annular body (12) interpositioned between said second compartment (9) and said second fluid space (4), said annular body (12) being distinct from said housing body (2).

According to a further embodiment, in each of the previous embodiments said annular body (12) is also distinct from said cap (8).

According to a further embodiment, in each of the previous embodiments said annular body (12) is interpositioned in contact between said sealing element (6) and said housing body (2).

According to a further embodiment of the invention a third separation device comprises: a housing body (2); a first fluid space (3) in said housing body (2); a second fluid space (4) situated between said first fluid space (3) and said housing body (2); a membrane separation element (5) which separates said first fluid space (3) from said second fluid space (4); at least one sealing element (6) arranged at an end of said separation element (5); at least a first compartment (7) in fluid communication with said first fluid space (3); at least a second compartment (9) in fluid communication with said second fluid space (4) and fluidly isolated from said first compartment (7); at least an annular body (12) interpositioned between said second compartment (9) and said second fluid space (4), at least a part of said annular body (12) radially containing a non-occupied zone of said second fluid space (4), where radially is intended to mean with reference to a longitudinal axis of said membrane separation element.

According to a further embodiment of the invention a fourth separation device comprises: a housing body (2); a first fluid space (3) in said housing body (2); a second fluid space (4) situated between said first fluid space (3) and said housing body (2); a membrane separation element (5) which separates said first fluid space (3) from said second fluid space (4); at least one sealing element (6) arranged at an end of said separation element (5); at least a first compartment (7) in fluid communication with said first fluid space (3), said sealing element (6) being interpositioned between said first compartment (7) and said second fluid space (4); at least a first cap (8) which at least partly delimits said first compartment (7) and which is contactingly coupled with said housing body (2); at least a second compartment (9) fluidly connected to said second fluid space (4) and fluidly isolated from said first compartment (7), said first cap (8) being interpositioned between said second compartment (9) and said first compartment (7); at least a second cap (10) which at least partly delimits said second compartment (9).

According to an embodiment of the invention a support for a separation device comprises: a first connection element (21) having a central fluid access port (22) and a peripheral fluid access port (23) which surrounds said central fluid access port (22); a second connection element (37) having at least a third fluid access port (38); said first connection element (21) and said second connection element (22) being able to assume at least a first operative closed position, in which a separation device (5) arranged between said first connection element (22) and said second connection element (23) in a work position is fluidly connected to all three of said fluid access ports, and at least a second operative open configuration, in which said separation device can be inserted into and disinserted from and between said first connection element (21) and said second connection element (22).

According to a further embodiment, said peripheral fluid access port (23) is internally delimited by a first wall, a first sealing element (25) being predisposed on said first wall to interact contactly with said separation device (5) in a work position.

According to a further embodiment, in the previous embodiment an additional sealing element (34) is predisposed on said first wall, which additional sealing element (34) is axially distanced from said first sealing element (25).

According to a further embodiment, in the previous embodiment at least a connection with a vent (35) is predisposed on said first wall, between said additional sealing element (34) and said first sealing element (25).

According to a further embodiment, in each of the four previous embodiments said peripheral fluid access port (23) is externally delimited by a second wall, on which a second sealing element (26) is predisposed.

According to a further embodiment, in each of the five previous embodiments said first connection element is made of at least two hollow parts, a first part (21 a) being at least partially contained internally of a second part (21 b), a first flud space (27) internal of said first hollow part communicating with said central access port (22), a second fluid space (28) defined between said first and said second hollow parts (21a and 21 b) being in communication with said peripheral access port (23).

According to a further embodiment, the previous embodiment comprises at least two reciprocally-distanced sealing elements (29 and 36) which operate between said first hollow part (21 a) and said second hollow part (21 b), a vent (37) being fluidly connected to a space comprised between said two sealing elements (29 and 36).

According to an embodiment of the invention, an apparatus for extracorporeal blood treatment comprises at least one hydraulic circuit for a treatment fluid and at least one support for a separation device destined during use to be fluidly connected with said hydraulic circuit, said support being made according to any one of the seven previous embodiments.

According to a further embodiment, the apparatus of the previous embodiment is predisposed to perform one or more of following treatments: hemodialysis, hemofiltration, hemodiafiltration, pure ultrafiltration, plasmapheresis.

Further characteristics and advantages of the present invention will better emerge from the detailed description which follows, of at least one embodiment of the invention, illustrated by way of non-limiting example in the accompanying figures of the drawings.

### 1.1.2 Brief Description of the Drawings

The following description is made with reference to the accompanying figures of the drawings, provided for indication and therefore non-limiting, in which:
figure 1 is a partial half-section of one end of a separation device made according to the invention, made along a section plane passing through the longitudinal axis of the device;
figure 2 shows the annular body of the device of figure 1 in section;
figure 3 shows a plan view of the annular body of figure 2;
figures from 4 to 7 show four different embodiments of the first connection element of a support for a separation device, made according to the invention;
figure 8 shows the second connection element of a support for a separation device, made according to the invention.

### 1.1.2.1.1 Detailed Description

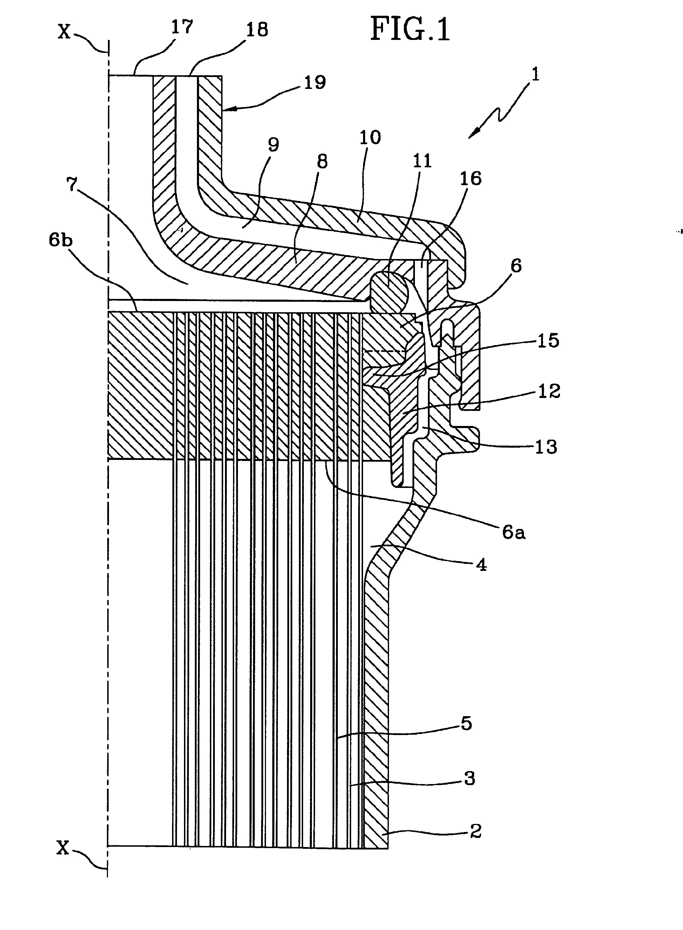

With reference to figures 1 to 3, 1 denotes in its entirety a separation device which, in the specific case, is usable in an apparatus for extracorporeal blood treatment. In particular the separation device 1 can be used as an ultrafilter in a machine (of known type and not illustrated) for on-line preparation of dialysis fluid and/or infusion fluid. The separation device 1 can also be used as a blood treatment unit (for example as a dialysis filter).

Figure 1 shows (in half-section) a first end of the separation device 1. Figure 1 only shows one half (right part) of the upper end of the separation device, the other half (left part) being specular thereto. In the illustrated embodiment the separation device 1 has a second end (not illustrated) opposite to the illustrated upper first end and specular thereto.

The separation device 1 comprises an elongate hollow housing body 2 which extends along a longitudinal axis x-x. The housing body 2 has an essentially cylindrical main part.

The separation device 1 comprises, internally of the housing body 2, a first fluid space 3 and a second fluid space 4 situated between the first fluid space 3 and the housing body 2.

A membrane separation element 5 separates the first fluid space 3 from the second fluid space 4. The membrane is semi-permeable.

The membrane separation element 5 is prevalently elongate along a longitudinal axis x-x.

The longitudinal axis of the separation element 5 is parallel to the longitudinal axis of the housing body 2.

The longitudinal axis of the separation element 5 coincides with the longitudinal axis of the housing body 2.

The membrane separation element 5 comprises a bundle of hollow fibres. The first fluid space 3 comprises a space internal of the hollow fibres. The second fluid space 4 is a space external of the hollow fibres.

A sealing element 6 is arranged at an end of the membrane separation element 5. A further sealing element (not illustrated) is arranged at the opposite end thereof.

An end of the separation element 5 is sunk into the sealing element 6. Similarly, the other end (not illustrated) of the separation element 5 is sunk into the other sealing element.

The membrane separation element 5 is fixed internally of the housing body 2 by means of the sealing element 6, as will be better explained herein below.

Each sealing element 6 is made of a plastic material (for example a polyurethane material).

The separation device 1 comprises a first compartment 7 in fluid connection with the first fluid space 3. At the non-illustrated end thereof, the separation device 1 comprises a further compartment, specular to the first compartment 7 of figure 1 and also in communication with the first fluid space 3.

The separation device 1 comprises a first cap 8, conformed and arranged to define the first compartment 7 together with the sealing element 6. At the non-illustrated end, the separation device 1 comprises a further first cap, specular to the first cap 8 of figure 1.

The separation device 1 comprises a second compartment 9 in fluid connection with the second fluid space 4. The second compartment 9 is fluidly isolated from the first compartment 7.

The separation device 1 further comprises a second cap 10 which is conformed and arranged so as to define the second compartment 9 together with the first cap 8.

At the non-illustrated end, the separation device 1 comprises another second compartment, specular to the other second compartment 9 of figure 1 and also in fluid connection with the second fluid space 4, and a further second cap, specular to the other second cap 10 of figure 1.

The sealing element 6 has an internal side 6a which is in fluid communication with the second fluid space 4, and an external side 6b which is in fluid communication with the first fluid space 3.

The sealing element 6 is essentially disc-shaped with an axis which is parallel to the longitudinal axis x-x of the membrane separation element 5.

The external side 6b of the sealing element 6 comprises a surface, which in the illustrated embodiment is flat but which could be concave or convex, facing in a direction which is essentially perpendicular to the longitudinal axis x-x of the membrane separation element 5.

The internal side 6a of the sealing element 6 comprises a surface, which in the illustrated embodiment is flat but which could be concave or convex, facing in a direction which is essentially perpendicular to the longitudinal axis x-x of the membrane separation element 5, in an opposite direction to the above-mentioned surface of the external side 6b.

The first cap 8 at least partially covers the external side 6b of the sealing element. In the illustrated embodiment the first cap 8 does not directly interact in contact with the external side 6b.

The second cap 10 at least partially covers the first cap 8.

The first cap 8 has an annular edge which is coupled to an annular edge of the housing body 2. The annular edge of the housing body 2, which is coupled to the first cap 8, is situated at an end of the housing body 2, i.e. at an axial annular end zone (with reference to the longitudinal axis x-x of the housing body 2) beyond which no other parts of the housing body 2 lie.

In the illustrated embodiment the first cap 8 is connected solidly, via a permanent and non-detachable attachment, to the housing body 2.

The second cap 10 is entirely arranged beyond the housing body 2, where "beyond" is taken to mean with reference to an axial direction parallel to the longitudinal axis x-x of the housing body 2.

The first cap 8 is in contact with the housing body 2 exclusively in a peripheral zone of the first cap 8. The peripheral zone is, in the illustrated embodiment, annular and wider and external with respect to the bundle of hollow fibres which make up the separation element 5.

The sealing element 6 is interpositioned between the first compartment 7 and the second fluid space 4. The sealing element 6 separates, with a fluid seal, the first compartment 7 from the second fluid space 4.

A sealing element 11, in effect an o-ring, is interpositioned between the first cap 8 and the sealing element 6. The sealing element 11 is annular. The sealing element 11 is operatively situated on a peripheral zone of the external side 6b of the sealing element 6.

The sealing element 11 fluid-sealedly separates the first compartment 7 from the second fluid space 4 and the second compartment 9.

The separation device 1 comprises an annular body 12 interpositioned fluidly between the second compartment 9 and the second fluid space 4.

The annular body 12 is provided with one or more openings 13, to fluidly connect the second compartment 9 with the second fluid space 4. In the illustrated embodiment a plurality of the fluid comunication openings 13 are provided.

The passage opening or openings 13 can be contiguous to the annular body 12 or can pass through the annular body 12.

The annular body 12 comprises a plurality of support feet 14, in the form of radial projections arranged on the periphery of the annular body and reciprocally distanced in a circumferential direction. When the annular body 12 is inserted in the separation device 1, it is arranged between the sealing element 6 and the housing body 2. In this position, between two adjacent support feet 14 there is a passage or channel, through which fluid connection is possible between the second compartment 9 and the second fluid space 4. The passages or channels realise, in the illustrated embodiment, the fluid communication openings 13.

The support feet 14 rest, in simple resting and detachable contact i.e. with no solid and permanent attachments, on one or more of the elements which are part of the casing of the separation device 1 (in the illustrated embodiment the casing comprises the housing body 2 and the end caps 8 and 10): in the illustrated embodiment the rest element is represented by the housing body 2.

The annular body 12 is of a closed annular shape.

The first cap 8 is coupled contactingly to the housing body 2.

The annular body 12, which is interpositioned fluidly between the second compartment 9 and the second fluid space 4, is distinct from the housing body 2. In other words, the annular body 12 is a separate element from the housing body 2, manufactured separately and united during assembly.

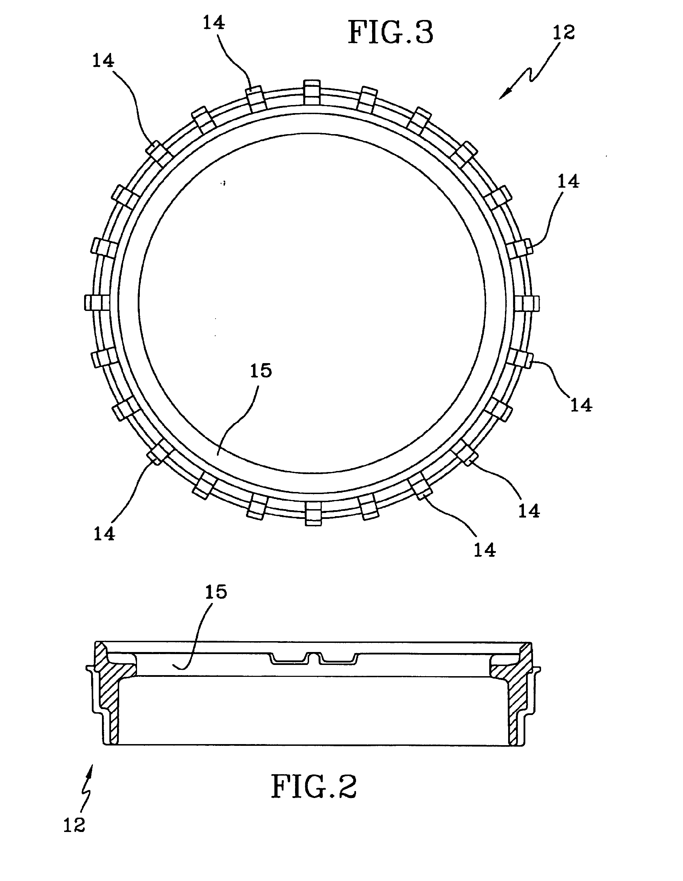

The annular body 12 can be structured and arranged as the support ring 40 of the embodiments (figures 2 to 7) described in US 2003/0029785 which is herein incorporated by reference.

The annular body 12 is interpositioned, in contact both with the internal and the external sides, between the sealing element 6 and the housing body 2.

The sealing element 6 is in direct contact with the annular body 12.

The annular body 12 has, on an internal side thereof, at least one projecting part 15 which projects radially internally and which is immersed into the material of the sealing element 6. The projecting part 15 forms at least one engagement zone which avoids or limits relative axial displacements, in both axial directions, between the annular body 12 and the sealing element 6. The annular body 12 can have, for instance, one or more of the following fitting elements which the support ring 40 of the filter 100 described in US 2003/0029785 is provided with: a plurality of lugs 42, a rim or shoulder 44, flanges 46 and steps 48 and 50.

The contact between the sealing element 6 and the annular body 12 is detachable, i.e. it is a simple contact arrangement with no solid and permanent attachment.

The sealing element 6 has a peripheral surface which is radially directed, with reference to a longitudinal axis x-x of the membrane separation element 5. The peripheral surface is embraced by the annular body 12, in a circumferential direction, in a detachable contact arrangement.

The peripheral surfaces of the sealing element 6 and the annular body 12 exhibit a reciprocal contact zone; this contact zone (detachable) is without undercuts in radial direction towards the periphery.

At least a part of the sealing element 6 is plate-shaped or disc-shaped and extends radially, where radially is taken to mean with reference to the longitudinal axis x-x of the separation element 5; this plate- or disc-shaped part has a peripheral surface facing radially, which is at least in partial contact with the annular body 12. The annular body 12 and the peripheral surface are in reciprocal contact at least at an axial undercut position.

The first cap 8 and the second cap 10 are coaxial. The first cap 8 and the second cap 10 are conformed as bodies of revolution about an axis of symmetry.

A part of the first cap 8, which is fluidly interpositioned between the second compartment 9 and the second fluid space 4, is provided with at least one hole 16 for fluid communication between the second compartment 9 and the second fluid space 4. The fluid communication hole 16 between the second compartment 9 and the second fluid space 4 is axially extended, with reference to a longitudinal axis x-x of the membrane separation element 5. In the illustrated embodiment the first cap 8 is provided with a plurality of holes 16 distributed in a circumferential direction.

The passage through the first cap 8 for enabling fluid communication between the second compartment 9 and the second fluid space 4 is arranged contiguously to the sealing element 11, immediately by the side thereof. The radial distance of the fluid communication passage from a central axis x-x of the first cap 8 is greater than the radial distance between the axis and the sealing element 11.

The first fluid space 3 and the second fluid space 4 are provided with a first access opening 17 and, respectively a second access opening 18. At least a part of the second access opening 18 surrounds at least a part of the first access opening 17. The second access opening 18 is annular and arranged about the first access opening 17. The first and second access openings 17 and 18 are coaxial to one another.

The separation device 1 has a connection port 19 provided with the coaxial first and second access openings 17 and 18.

The separation device 1 comprises, at the end thereof which is not illustrated, a connection port which is specular to the connection port 19 shown in figure 1.

At least a part of the annular body 12 radially surrounds the sealing element 6, where "radially" is taken to mean with reference to the longitudinal axis of the membrane separation element 5.

At least a part of the annular body 12 radially contains a non-occupied zone of the second fluid space 4, where radially is taken to mean with reference to the longitudinal axis of the membrane separation element 5.

At least a part of the annular body 12 is arranged beyond the second fluid space 4, where beyond is taken to mean with reference to the direction of the longitudinal axis x-x of the membrane separation element 5, i.e. the longitudinal axis of the bundle of hollow fibres.

At least a part of the annular body 12 radially surrounds a part of the sealing element 6 which sunkenly contains all or almost all or at least a majority of the hollow fibres of the bundle forming the membrane separation element 5.

The second cap 10 is coupled contactingly with the first cap 8.

The second cap 10 has on a periphery thereof an annular edge coupled to an annular edge of the first cap 8.

The second cap 10 is welded to the first cap 8.

The first cap 8 is interpositioned between the first compartment 7 and the second compartment 9.

The annular body 12 is distinct from both the housing body 2 and the first cap 8 and the second cap 10.

The second cap 10 interacts contactingly with the first cap 8, exclusively along a coupling zone arranged on a peripheral edge of the second cap 10. The coupling zone has a closed annular shape.

The coupling zone is arranged contiguously to the series of passage holes 16, distributed in circumferential direction on the first cap 8, for fluid communication between the second compartment 9 and the second fluid space 4; the coupling zone is situated externally of the above-cited series of passage holes 16.

The only contact zone between the first and the second caps 8 and 10 is situated at a predetermined distance from the longitudinal axis x-x of the membrane separation element 5 and/or the housing body 2. The distance is greater than the radial distance of the series of passage holes 16 from the longitudinal axis x-x.

The second compartment 9 develops between a peripheral zone, situated contiguously to the circumferential series of passage holes 16, and a central zone, located at the second access opening 18 in which the second compartment opens externally, with no break in continuity. In other embodiments (not illustrated) contact elements are provided between the first and the second caps 8 and 10; such contact elements are situated along the fluid path, internally of the second compartment, comprised between the holes 16 on the first cap 8 and the externally-opening annular opening 18.

The annular support body 12, which keeps the sealing element 6 in position, enables a detachment of the material forming the sealing element 6 - a detachment caused, for example, by a heat stress subsequent to a disinfection or sterilization operation - without the risk of causing fractures or other deformations or imperfections in the material of the sealing element 6.

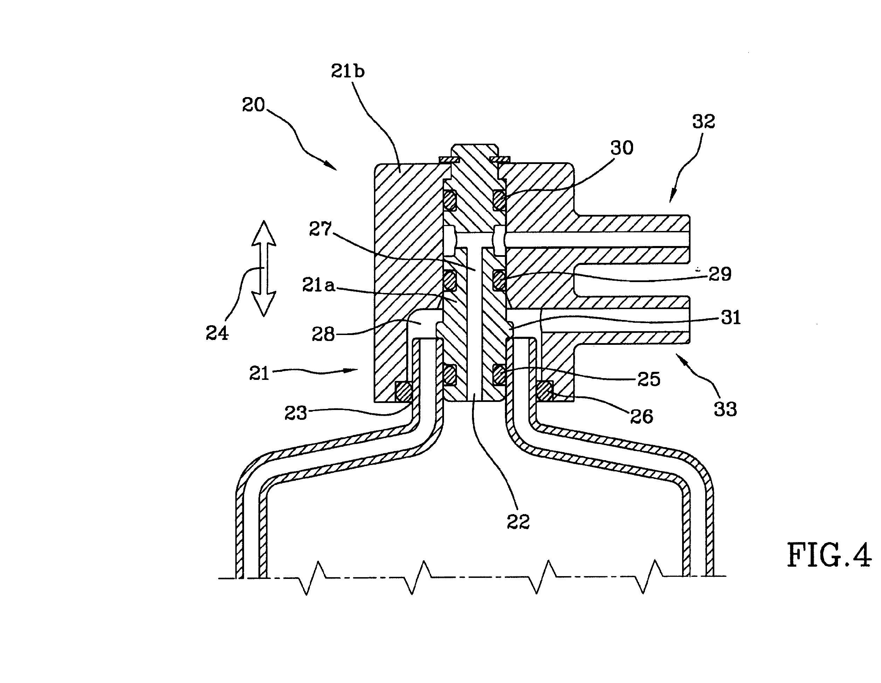

With reference to figure 4, 20 denotes a support for a separation device. The support 20 is a part, in the illustrated embodiment, of a machine for extracorporeal blood treatment (not illustrated), such as for example a dialysis machine able to perform on-line dialysis fluid and/or infusion fluid preparation.

The apparatus for extra-corporeal blood treatment comprises at least one hydraulic circuit (of known type) for a treatment fluid. The support 20 is destined, during use, for fluid connection at least with the hydraulic circuit of the apparatus.

The support 20 is couplable with the separation device 1 of the preceding figures.

The support 20 comprises an annular first connection element 21, having a central fluid access port 22 and an annular peripheral fluid access port 23, which surrounds the central access port 22.

The support 20 further comprises a second connection element having at least a third fluid access port.

In the illustrated embodiment the second connection element (not illustrated) is specular to the first connection element 21. The second connection element comprises a fourth fluid access port. The third and fourth fluid access ports are specular to the central access port 22 and, respectively, to the peripheral access port 23.

The first and the second connection elements can assume at least a closed first operative configuration, in which the separation device arranged between the two connection elements is fluidly connected in the work position to the three or four fluid access ports (figure 4), and at least an open second operative position, in which the separation device can be inserted and disinserted between the two connection elements.

Figure 4 shows only the upper part of the closed configuration, the lower part being specular to the upper part.

In the illustrated embodiment, the displacement between the first and second working configurations, opening and closing, comprises a nearing and distancing movement of the two connection elements. The movement can comprise a linear translation in a direction indicated by the arrow 24. Possibly only one of the connection elements can be displaced, or both. The means for moving the connection element or elements can be, for example, any known-type translation device, or known systems for removable gripping of filters, cartridges or other replaceable devices, already used, for example in dialysis machines.

The annular fluid access port 23 is internally delimited by a first wall.

A first annular sealing element 25 is predisposed on the first wall, and contactingly interacts with the separation device in the working position.

The internal first wall is essentially cylindrical in shape.

The annular fluid access port 23 is externally delimited by a second wall, on which a second sealing element 26 is arranged. The external second wall is also essentially cylindrical and coaxial to the first wall.

The first connection element 21 is made of at least two hollow parts. An internal first part 21 a is at least partly contained internally of an external second part 21 b.

A first fluid space 27, internal of the first hollow part 21 a, communicates with the central access port 22.

A second fluid space 28, defined between the first and the second hollow part, communicates with the annular access port 23.

The support 20 comprises at least two sealing elements 29 and 30, distanced one from another and operating between the first and the second hollow parts 21 a and 21 b.

One of these sealing elements, denoted by 29, is predisposed between the first and second fluid spaces 27 and 28. The other sealing element 30 isolates the first fluid space 27 from the outside.

The internal wall exhibits a projection 31 which acts as a striker surface against which, during the insertion stage, the separation device interacts strikingly.

The first and second fluid spaces 27 and 28 terminate with two respective fluid connection mouths 32 and 33.

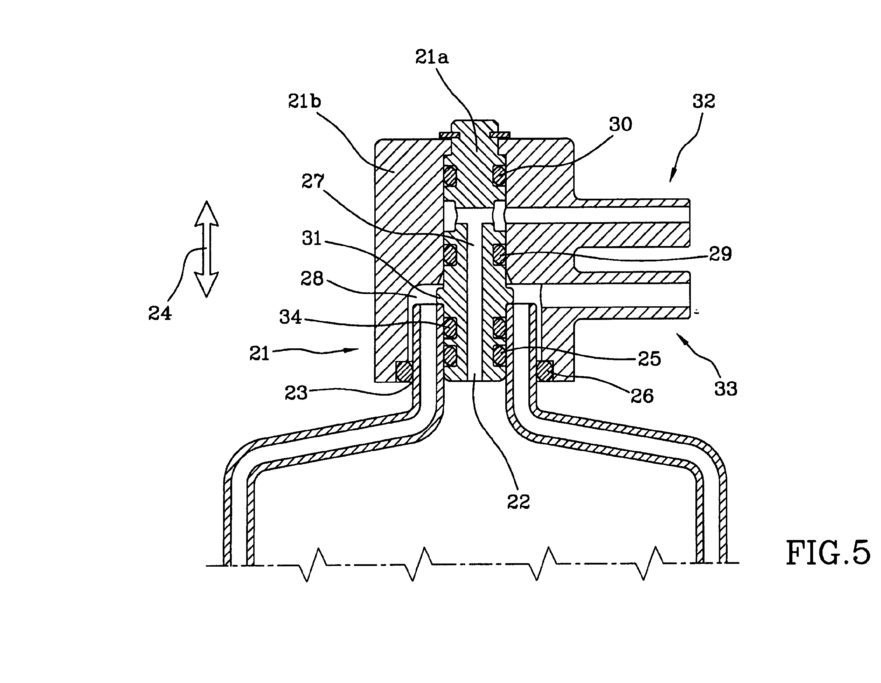

With reference to the illustrated embodiment of figure 5 (in which the elements similar to those in figure 4 are denoted with the same numbers), an additional annular sealing element 34 is predisposed on the first wall, axially distanced from the first annular sealing element 25 and operating on the separation device during use.

In this embodiment, thanks to the additional annular sealing element 34, the risk of leakage is lower.

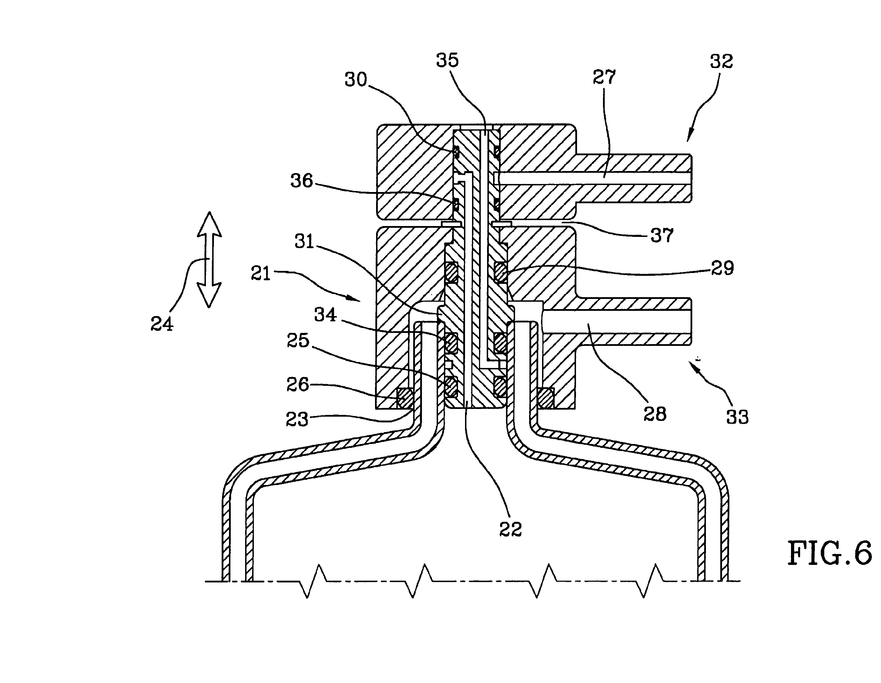

With reference to the embodiment of figure 6, on the first wall, between the additional annular sealing element 34 and the first sealing element 25, there is at least one connection with a vent 35.

The vent connection comprises a channel afforded in the connection element 21.

A further sealing element 36 operates between the two parts 21 a and 21 b of the first connection element 21 and in the separation zone between the first and second fluid space 27 and 28. A connection with a vent 37 is prediposed between the two sealing elements 29 and 36. The vent 37 is obtained in the illustrated embodiemnt by making the second external part 21 b in two parts.

In the embodiment of figure 6, all the seals, both the static seals 29, 30 and 36 and the dynamic seals 25 and 34, are during operation interpositioned between a fluid space internal of the support and the outside environment.

Therefore, if a fluid space internal of the support is at a greater pressure than ambient pressure, and if any one of the seals, whether static or dynamic, of the support is not working efficiently, there is a leakage of fluid from the fluid space in overpressure towards the outside, with a consequent pressure drop in the fluid space. Thus it is possible, by placing one or more of the fluid spaces internal of the support in overpressure, to make a very simple check on the functioning of the sealing elements.

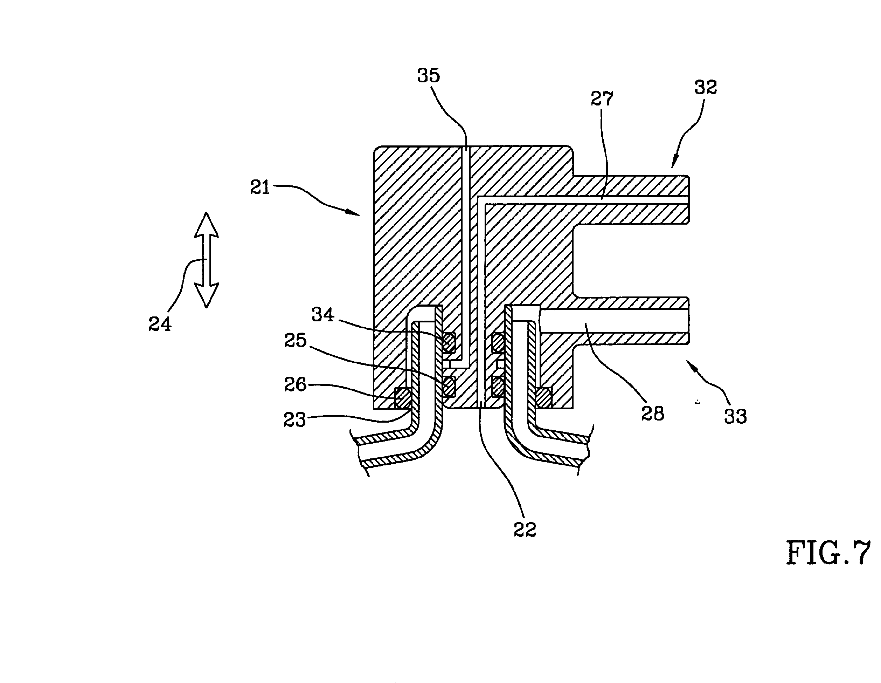

With reference to the embodiment of figure 7, the connection element 21 is made in a single piece. No static sealing elements are included.

Furthermore, in this embodiment the central access opening of the separation device is more external with respect to the annular peripheral access opening. The separation device has a connection port, with two coaxial access openings, which connection port has a central element (solidly constrained to the first cap 8) which is more projecting, in a radial direction, with respect to a peripheral annular element (solidly constrained to the second cap 10).

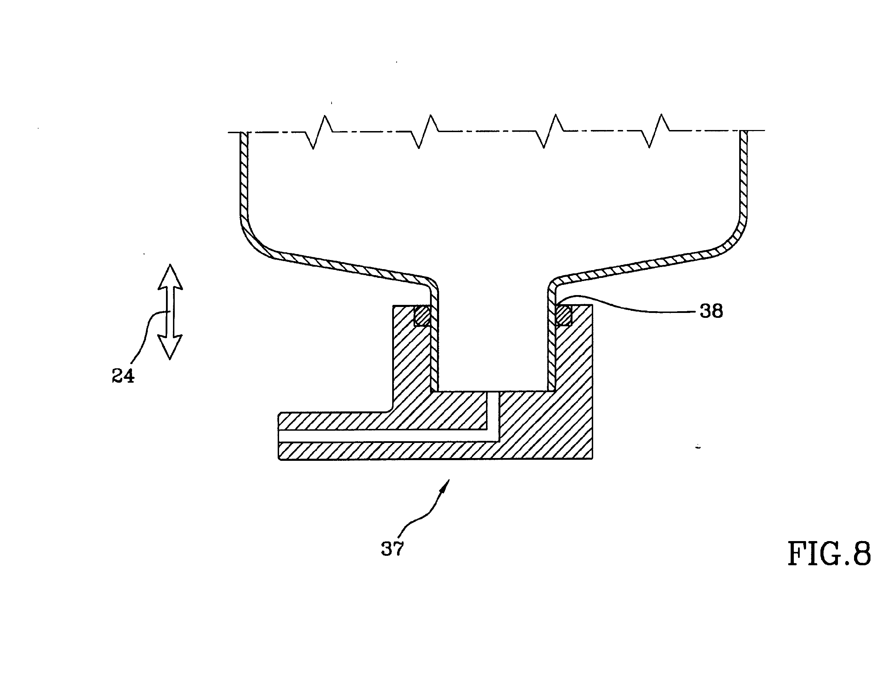

Figure 8 shows a specific embodiment of a second connection element 37, having one fluid access opening 38 only.

In this embodiment the support is suitable for keeping a separation device in position when the separation device has only three fluid access openings, such as for example an ultrafilter of a dialysis machine.

## Claims

1. A support for a separation device, comprising:
a first connection element (21) having a central fluid access port (22) and a peripheral fluid access port (23) which surrounds said central fluid access port (22);
a second connection element (37) having at least a third fluid access port (38); said first connection element (21) and said second connection element (22) being able to assume at least a first operative closed position, in which a separation device (5) arranged between said first connection element (22) and said second connection element (23) in a work position is fluidly connected to all three of said fluid access ports, and at least a second operative open configuration, in which said separation device can be inserted into and disinserted from between said first connection element (21) and said second connection element (22).

2. The support of claim 1, wherein said peripheral fluid access port (23) is internally delimited by a first wall, a first sealing element (25) being predisposed on said first wall to contactly interact with said separation device (5) in a work position.

3. The support of claim 2, wherein an additional sealing element (34) is predisposed on said first wall, which additional sealing element (34) is axially distanced from said first sealing element (25).

4. The support of claim 3, wherein at least a connection with a vent (35) is predisposed on said first wall, between said additional sealing element (34) and said first sealing element (25).

5. The support of any one of claims from 1 to 4, wherein said peripheral fluid access port (23) is externally delimited by a second wall, on which a second sealing element (26) is predisposed.

6. The support of any one of claims from 1 to 5, wherein said first connection element is made of at least two hollow parts, a first part (21 a) being at least partially contained internally of a second part (21 b), a first fluid space (27) internal of said first hollow part communicating with said central access port (22), a second fluid space (28) defined between said first and said second hollow parts (21 a and 21 b) being in communication with said peripheral access port (23).

7. The support of claim 6, comprising at least two reciprocally-distanced sealing elements (29 and 36) which operate between said first hollow part (21 a) and said second hollow part (21 b), a vent being fluidly connected to a space comprised between said two sealing elements (29 and 36).

8. An apparatus for extracorporeal blood treatment, comprising at least one hydraulic circuit for a treatment fluid and at least one support (20) for a separation device destined during use to be fluidly connected with said hydraulic circuit, said support being made according to any one of claims from 1 to 7, optionally wherein said support comprises a second connection element which is specular to the first connection element.

9. An apparatus for extracorporeal blood treatment according to claim 8, further comprising a separation device coupled to the support (20), said separation device comprising:
a housing body (2);
a first fluid space (3) in said housing body;
a second fluid space (4) situated between said first fluid space (3) and said housing body (2);
a membrane separation element (5) separating said first fluid space (3) from said second fluid space (4);
at least one sealing element (6) arranged at an end of said membrane separation element (5);
at least one first compartment (7) fluidly connected to said first fluid space (3);
at least one second compartment (9) fluidly connected with said second fluid space (4) and fluidly isolated from said first compartment (7); the separation device (5) being arranged between said first connection element (22) and said second connection element (23) in a work position where it is fluidly connected to all three of said fluid access ports.

10. The apparatus for extracorporeal blood treatment of claim 9, wherein:
said first compartment (7) is provided with a first access opening (17);
said second compartment (9) is provided with a second access opening (18);
at least a part of said second access opening (18) surrounds at least a part of said first access opening (17).

11. The apparatus for extracorporeal blood treatment of claim 9 or 10, comprising at least an annular body (12) in interposition between said second compartment (9) and said second fluid space (4).

12. The apparatus for extracorporeal blood treatment of claim 11, comprising at least one passage (13), contiguous to said annular body (12), or passing through said annular body (12), for fluidly connecting said second compartment (9) with said second fluid space (4), wherein said at least one passage (13) is afforded in said annular body (12).

13. The apparatus for extracorporeal blood treatment of any one of the claims 11 to 12, wherein said sealing element (6) has a peripheral surface which is radially directed, with reference to a longitudinal axis (x-x) of said membrane separation element (5), and which is circumferentially contactingly embraced by said annular body (12) in fluid interposition between said second compartment (9) and said second fluid space (4), and wherein said annular body (12) has a closed annular shape, optionally wherein said peripheral surface of said sealing element (6) and said annular body (12) have a reciprocal contact zone, which is free of undercuts in a radial direction towards the periphery.

14. The apparatus for extracorporeal blood treatment of any one of the claims 9 to 13, wherein said sealing element (6) is in contact with said annular body (12) and wherein said annular body (12) is distinct from said housing body (2) and is interpositioned contactingly between said sealing element (6) and said housing body (2).

15. The apparatus for extracorporeal blood treatment of any one of the claims 9 to 14, wherein at least a part of said sealing element (6) is a radially extended plate, with reference to a longitudinal axis (x-x) of said membrane separation element (5), said plate of said sealing element (6) having a radially-directed peripheral surface, which is at least partially in contact with said annular body (12) fluidly interpositioned between said second compartment (9) and said second fluid space (4), and wherein said annular body (12) and said peripheral surface are in reciprocal contact at least at an axial undercut (15), optionally wherein at least a part of said annular body (12) radially contains a zone which is not occupied by said second fluid space, where radially is intended to mean with reference to a longitudinal axis (x-x) of said membrane separation element (5).

16. The apparatus for extracorporeal blood treatment of any one of the claims 9 to 15, comprising:
at least a first cap (8) conformed and arranged in order to at least partly define said first compartment (7) together with said sealing element (6); said first cap (8) having a terminal wall having an external edge which internally delimits said second access opening (18), and an internal edge which externally delimits said first access opening (17); said first cap (8) being in contact with said housing body (2); said first cap (8) having an annular edge which is coupled to an annular edge of said housing body (2); said first cap (8) being solidly connected to said housing body (2); said first cap (8) at least partly covering an external side (6b) of said sealing element (6); a part of said first cap (8), which is interpositioned between said second compartment (9) and said second fluid space (4), being provided with at least one hole (16) for fluid communication between said second compartment (9) and said second fluid space (4); said at least one hole (16) extending prevalently in an axial direction, with reference to a longitudinal axis (x-x) of said membrane separation element (5); and
at least a second cap (10) conformed and arranged in such a way as to at least partially define said second compartment (9) in collaboration with said first cap (8); said second cap (10) at least partly covering said first cap (8); said second cap (10) being coaxial to said first cap (8).
